# EUROPÄISCHE PATENTANMELDUNG

(11) **EP 1 543 813 A1**
(43) Veröffentlichungstag der Anmeldung: **22.06.2005**
(21) Anmeldenummer: 04015070.8
(22) Anmeldetag: 26.06.2004
(51) Int. Cl.: A61K 7/021, A61K 7/48, C09D 13/00

(54) **Kosmetische Feststoffmine**

(30) Priorität: 19.12.2003 DE 20319911 U
(71) Anmelder: Faber-Castell AG, 90546 Stein (DE)
(72) Erfinder: Appel, Reiner, 90522 Oberasbach (DE); Haller, Martin, 90451 Nürnberg (DE); Lugert, Gerhard, Dr., 90431 Nürnberg (DE); Rechinger, Michael, Dr., 90431 Nürnberg (DE)
(74) Vertreter: Mörtel & Höfner

(57) **Zusammenfassung**

Die Erfindung betrifft eine von einem Kunststoff ummantelte kosmetische Feststoffmine mit einer bei 20°C flüssigen lipophilen Phase, welche mit einem Anteil von wenigstens 25 % vorhanden und vollständig oder zumindest zu einem überwiegenden Teil von Fettalkoholen mit 16 bis 20 C-Atomen gebildet ist. Die Feststoffmine ist mit einem Kunststoff auf der Basis eines Celluloseesters ummantelt.

## Beschreibung

Die Erfindung betrifft eine mit Kunststoff ummantelte kosmetische Feststoffmine mit einer bei Raum- bzw. Umgebungstemperatur flüssigen lipophilen Phase. Diese ist mit einer bei Umgebungstemperatur festen Grundmasse innig vermischt. Als flüssige Phase werden herkömmlicherweise mineralische Öle wie Paraffinöl mit hohen Anteilen unverzweigter Ketten und Fettsäureester wie Tetradecansäure-isopropylester (Iso-propylmyristat) verwendet. Die feste Phase bilden vielfach Wachse und Fettsäurederivate. Die oft in hohen Anteilen vorhandene flüssige lipophile Phase bewirkt eine Minenkonsistenz, die einen weichen Abstrich auf der Haut ermöglicht und verleiht den geschminkten Hautpartien einen Glanz.

Die flüssige lipophile Phase, insbesondere wenn sie in hohen Anteilen vorhanden ist, bewirkt eine Verringerung der Temperaturstabilität der kosmetischen Mine. Bei erhöhten Temperaturen, etwa bei Aufbewahrung der Mine in beheizten Räumen, neigt die Mine zum Ausölen, d.h. die mit der festen Phase vorher homogen vermischte flüssige lipophile Phase trennt sich ab, es kann zur Bildung von Tropfen an der Minenspitze kommen, die zu Verschmutzungen führen und bei der Applikation stören können. In gewissem Ausmaß kann hier Abhilfe geschaffen werden, wenn eine Minenmatrix mit erhöhtem Rückhaltevermögen für die flüssige lipophile Phase, also etwa für Öle, verwendet wird. Geeignet sind vor allem mikrokristalline Wachse. Auch wird den geschilderten Effekten entgegengewirkt, indem Minenummantelungen verwendet werden, die zumindest einen Teil der ausgeschwitzten flüssigen Phase aufnehmen. Werkstoffe, die dies leisten können sind solche mit einer mikroporösen Struktur, etwa Holz oder Kunststoffe, denen bei der Herstellung Porenbildner zugesetzt wurden. Flüssigkeit kann jedoch nur dann aufgenommen werden, wenn eine offenporige Struktur vorliegt, d.h. wenn sich die Mikroporen in die Innenfläche der Minenummantelung öffnen und untereinander in Verbindung stehen. Letzteres kann die Permeationsdichtigkeit der Ummantelung verringern, was im Hinblick auf ein dadurch bedingtes Verdunsten flüchtiger Minenbestandteile unerwünscht ist. Die Mikroporen erschweren außerdem die Herstellung klar durchsichtiger Ummantelungen oder machen dies gar unmöglich. Eine weitere Möglichkeit, dem geschilderten Effekt entgegen zu wirken, besteht darin, der Ummantelung Füllstoffe mit einer offenporigen Struktur beizumengen. Solche Füllstoffe haben aber erhebliche Auswirkungen auf das optische Erscheinungsbild der Ummantelung und beeinflussen außerdem deren chemische und mechanische Eigenschaften.

Aufgabe der Erfindung ist es, eine mit Kunststoff ummantelte Kosmetikmine mit einem hohen Anteil an einer bei Raumtemperatur flüssigen lipophilen Phase vorzuschlagen, bei der das Ausölen auf eine alternative Weise vermieden ist.

Diese Aufgabe wird nach Anspruch 1 durch eine Kosmetikmine gelöst. Der Grundgedanke besteht dabei darin, dass die mit einem hohen Anteil, mit mindestens 25 %, vorhandene lipophile Phase vollständig oder zumindest zu einem überwiegenden Teil von einer chemischen Substanz gebildet ist, die sich in dem Kunststoff der Ummantelung löst. Damit ist ganz allgemein der Vorteil verbunden, dass ein Kunststoff eingesetzt werden kann, der ein ausreichendes Aufnahmevermögen für die flüssige lipophile Phase aufweist, der aber weder Mikroporen noch Füllstoffe enthalten muss. Ersteres birgt die Gefahr einer Permeation flüchtiger Minenbestandteile in sich. Letzteres beeinträchtigt die Transparenz etwa von Kunststoffen auf der Basis von weichgemachten Celluloseestern. Für diese Kunststoffgruppe, insbesondere Cellulosepropionat (CP), Celluloseacetat (CA) und Celluloseacetobutyrat (CAB), hat sich überraschenderweise gezeigt, dass sich Fettalkohole mit 16 bis 20 C-Atomen, insbesondere Oleylalkohol und/oder Isostearylalkohol als lipophile flüssige Phase oder als deren Hauptbestandteil eignen. Mit diesen Substanzen lassen sich Minenmassen mit einer Temperaturbeständigkeit bis wenigstens 40 °C herstellen. Sie sind darüber hinaus in der Lage, sich etwa nach Art eines primären Weichmachers im Celluloseester zu lösen oder - bei umgekehrter Sichtweise - diesen zu lösen. So lange die lipophile flüssige Phase noch homogen mit der Minenmatrix vermischt ist, ist die in den Kunststoff übertretende Menge vernachlässigbar gering. Erst wenn die lipophile Phase aufgrund einer Erwärmung aus der Minenmasse austritt und an der Minenoberfläche einen Flüssigkeitsfilm bildet, dringt dieser in die der Minenoberfläche nahen Materialschichten der Ummantelung ein.

Minen mit einer bei Raumtemperatur ausreichend festen Konsistenz werden erhalten, wenn der Anteil an Fettalkohol im Bereich von 25 % bis 65 % liegt und ein Anteil an Wachs und/oder Fettsäurederivat von 5 % bis 35 % eingehalten wird. Solche Minen vertragen Temperaturen bis etwa 50 °C. Die Lösefähigkeit insbesondere gesättigter verzweigter Fettalkohole, speziell von Isostearylalkohol wird gesteigert, wenn der Minenmasse Adipin- und/oder Zitronensäureester (z.B. Di-n-butyladipat = Cetiol B oder Acetyl-tributylcitrat), bekannt als Weichmacher für Celluloseester, beigemengt werden. Diese Stoffe verbessern auch die thermische Stabilität der Minenmasse unabhängig von der Ölaufnahmefähigkeit der Ummantelung. Eine Stabilisierung bzw. eine Verbesserung des Ölrückhaltevermögens wird, wie oben bereits erwähnt, durch mikrokristalline Wachse (Paraffinwachse) aber auch durch hydriertes Rizinusöl erreicht. Die erfindungsgemäßen Formulierungen zeichnen sich außerdem durch geringe Füllstoffanteile (organische oder anorganische Füllstoffe) aus, oder es kann auf Füllstoffe ganz verzichtet werden. Der Celluloseester der Ummantelung weist vorzugsweise einen Weichmacheranteil von 5 bis 25 %, insbesondere von 18 bis 22 % auf. Damit ist eine ausreichende mechanische Stabilität, zugleich aber auch eine gute Spitzbarkeit und ein ausreichendes Aufnahmevermögen für eine sich aus der Minenmasse abscheidende lipophile Phase gegeben.

Zur Herstellung einer ummantelten Kosmetikmine bzw. eines eine Kunststoffummantelung aufweisenden Stiftes werden die homogenisierten und bei 70°C bis 100°C geschmolzenen Minenmassen direkt in eine röhrenförmige Kunststoffummantelung vergossen oder es werden nach dem Vermischen der Rohmaterialien Minenstränge extrudiert und in eine Kunststoffummantelung eingebracht.

### Basisrezeptur:

- Ungesättigter unverzweigter und/oder gesättigter, verzweigter Fettalkohol: 25 - 65 %
- Wachse und bei 40°C feste Fettsäurederivate Adipinsäure- und/oder Zitronensäureester: 5 - 35 % 0 - 12 %
- Pigmente: 0 - 30 %
- Füllstoffe: 0 - 20 %
- Additive wie Verdicker, Antioxidantien, Konservierungsmittel, Emulgatoren u.a.: 2 - 15 %

| Beispiel 1 | |
|---|---|
| Hydrogeniertes Rizinusöl | 10,00 % |
| Stearinsäure | 5,00 % |
| Carnaubawachs | 3,50 % |
| Oleylalkohol (Novol) | 30,00 % |
| Isostearylalkohol (CAS-Nr. 27458-93-1) | 20,00 % |
| Acetyl-tributylcitrat (CAS-Nr. 77-90-7) | 2,00 % |
| Verdicker (Bentone 27) | 2,45 % |
| Konservierungsmittel (Sensiva SC50) | 0,33 % |
| Antioxidans (Ascorbylpalmitat) | 0,02 % |
| Füllstoff (Talkum) | 8,00 % |
| Parfum | 0,50 % |
| Pigmente: Titandioxid | 4,00 % |
| Cloisonne Red | 14,20 % |

| Beispiel 2 | |
|---|---|
| Oleylalkohol (Novol) | 54,38 % |
| Mikrokristallines Wachs (CAS-Nr. 63231-60-7) | 11,89 % |
| Candelilla Wachs | 1,70 % |
| Füllstoffe (Spherica) | 3,40 % |
| Covabead PMMA | 3,40 % |
| CAB-O-SIL | 1,13 % |
| Pigmente: Cloisonne Red | 7,36 % |
| Timiron Diamond Cluster MP 149 | 6,80 % |
| Timiron Supersheen MP 1001 | 5,66 % |
| Timica Brilliant White | 2,04 % |
| Cloisonne Gold | 0,68 % |
| Additive: Bentone 27V | 1,13 % |
| Arconate HP | 0,37 % |
| Decanox MTS 70 | 0,05 % |
| Ascorbylpalmitat | 0,01 % |

| Beispiel 3 | |
|---|---|
| Isostearylalkohol (CAS-Nr. 27458-93-1) | 40,49 % |
| Mikrokristallines Wachs (CAS-Nr. 63231-60-7) | 15,75 % |
| Candelilla Wachs | 2,25 % |
| Kohlensäureester (Cetiol CC) | 11,25 % |
| Pigmente: EA 1156 | 3,37% |
| C 47-060 | 0,17 % |
| C 33-5000 | 1,12 % |
| Cloisonne Gold | 0,56 % |
| Timica Brilliant White | 6,75 % |
| Timiron Gold MP 127 | 9,00 % |
| Additive: Cetiol B | 4,50 % |
| Decanox MTS 70 | 3,37 % |
| Ascorbylpalmitat | 1,12 % |
| Bentone 27V | 0,06 % |
| Arconate HP | 0,02 % |
| Elestab CPN | 0,11 % |
| Sensiva SC50 | 0,11 % |

| Beispiel 4 | |
|---|---|
| Isostearylalkohol (CAS-Nr. 27458-93-1) | 38,71 % |
| Mikrokristallines Wachs (CAS-Nr. 63231-60-7) | 14,65 % |
| 1-Propylen, 2-Methyl-, homopolymer | |
| (C4H8)x Polyisobutylene (Rewopal PIB 1000) | 13,60 % |
| Candelilla Wachs | 2,09 % |
| Pigmente: C 19-003 | 0,42 % |
| C 33-5000 | 0,42 % |
| Cloisonne Red | 13,08 % |
| Flamenco Superpearl | 4,19 % |
| Cloisonne Orange | 4,19 % |
| Additive: Cetiol B | 6,28 % |
| Decanox MTS 70 | 0,06 % |
| Ascorbylpalmitat | 0,02 % |
| Bentone 27V | 1,57 % |
| Arconate HP | 0,52 % |
| Elestab CPN | 0,10 % |
| Sensiva SC50 | 0,10 % |

Verzeichnis von Rohmateriallieferanten:
- Bentone 27V:: Elementis, B-9000 Gent
- Sensiva SC50:: Schülke & Mayr, D-22851 Norderstedt
- Cloisonne Red:: Engelhard, USA Iselin. NJ. 08830
- Cloisonne Gold:: Engelhard, USA Iselin. NJ. 08830
- Cloisonne Orange:: Engelhard, USA Iselin. NJ. 08830
- Timica Brilliant White:: Engelhard, USA Iselin. NJ. 08830
- Flamenco Superpearl:: Engelhard, USA Iselin. NJ. 08830

- Spherica:: Ikeda, JAP, Tokyo 100-0005
- Cetiol B:: Cognis, D-40551 Düsseldorf
- Covabead PMMA:: LCW, F-95310 Saint-QuenI'aumone
- CAB-O-SIL TS 610:: Cabot, D-63457 Hanau
- C 47-060:: Sun Chemical, USC Cincinnati, OH 45232
- C 33-5000:: Sun Chemical, USC Cincinnati, OH 45232
- C 19-003:: Sun Chemical, USC Cincinnati, OH 45232
- Decanox MTS 70:: ADM, USA Decatur, IL 62526
- Ascorbylpalmitat:: Hoffman La Roche, CH-4070 Basel
- EA 1156:: Color Techniques, USA South Plainfield NJ
- Timiron Gold MP 127:: Merck, D-64271 Darmstadt
- Timiron Diamond Cluster MP149:: Merck, D-64271 Darmstadt
- Timiron Supersheen MP-1001:: Merck, D-64271 Darmstadt
- Elestab CPN:: Laboratoires, F-54425 Pulnoy
- Arconate HP:: Lyondell Chemie, NL-3014 Rot terdam
- Rewopal PIB 1000:: WITICO Surfactants GmbH, D-36396 Steinau
- Novol:: Croda, D-41334 Nettetal

Alle Prozentangaben sind Gewichtsprozent!

## Patentansprüche

1. Von einem Kunststoff ummantelte kosmetische Feststoffmine mit einer bei 20°C flüssigen lipophilen Phase
**gekennzeichnet durch**
eine mit einem Anteil von wenigstens 25 % vorhandene lipophile Phase, die vollständig oder zumindest zu einem überwiegenden Teil von Fettalkoholen mit 16 bis 20 C-Atomen gebildet ist, und einem Kunststoff auf der Basis eines Celluloseesters.

2. Feststoffmine nach Anspruch 1,
**dadurch gekennzeichnet,**
**dass** Oleylalkohol enthalten ist.

3. Feststoffmine nach Anspruch 1,
**dadurch gekennzeichnet,**
**dass** Isostearylalkohol enthalten ist.

4. Feststoffmine nach Anspruch 1, 2 oder 3,
**gekennzeichnet durch**
einen Fettalkohol-Anteil von 25 % bis 65 %.

5. Feststoffmine nach einem der Ansprüche 1 bis 4,
**dadurch gekennzeichnet,**
**dass** ein Adipinsäure- und/oder ein Zitronensäureester enthalten ist.

6. Feststoffmine nach Anspruch 5,
**gekennzeichnet durch**
einen Estergehalt von 0,1 % bis 12 %.

7. Feststoffmine nach einem der Ansprüche 1 bis 6,
**dadurch gekennzeichnet,**
**dass** wenigstens ein Wachs und/oder wenigstens ein Fettsäurederivat enthalten sind, welche bei 20 °C fest sind.

8. Feststoffmine nach Anspruch 7,
**dadurch gekennzeichnet,**
**dass** ein mikrokristallines Wachs enthalten ist.

9. Feststoffmine nach Anspruch 7 oder 8,
**gekennzeichnet durch**
einen Anteil an Wachs und/oder Fettsäure von 5 % bis 35 %.

10. Feststoffmine nach einem der Ansprüche 1 bis 11,
**dadurch gekennzeichnet,**
**dass** sie mit einem Celluloseester aus der Gruppe Celluloseacetat, Cellulosepropionat und Celluloseacetobutyrat ummantelt ist.

11. Feststoffmine nach einem der Ansprüche 1 bis 10,
**gekennzeichnet durch**,
einen Weichmacheranteil von 5 bis 25 %.

12. Feststoffmine nach Anspruch 11,
**gekennzeichnet durch**,
einen Weichmacheranteil von 18 bis 22 %.
